# EUROPEAN PATENT SPECIFICATION

(11) **EP 2 276 836 B1**
(45) Date of publication and mention of the grant of the patent: **18.01.2017**
(21) Application number: 09742382.6
(22) Date of filing: 28.04.2009
(51) Int. Cl.: C12N 11/14, C07K 17/14, C09D 5/16, C09D 189/00, B64C 21/10, B64D 15/00

(54) **SURFACES WITH IMMOBILIZED ENZYMES AND ANTI-ICING PROTEINS**
OBERFLÄCHEN MIT IMMOBILISIERTEN ENZYMEN UND ENTEISUNGSPROTEINEN
SURFACES PRÉSENTANT DES ENZYMES ET DES PROTÉINES ANTIGEL IMMOBILISÉES

(30) Priority: 09.05.2008 GB 0808350
(43) Date of publication of application: 26.01.2011
(73) Proprietor: Airbus Operations Limited, Bristol BS34 7PA (GB)
(72) Inventor: BAUER, Karin, 81663 Munchen (DE); BOLZMACHER, Christian, 81663 Munchen (DE); FRIEDBERGER, Alois, 81663 Munchen (DE); REIDT, Ulrich, 81663 Munchen (DE)
(74) Representative: Haslam, Simon David
(86) International application number: PCT/GB2009/050425
(87) International publication number: WO 2009/136186

(56) References cited:
- EP-A1- 1 661 955
- WO-A1-96/30459
- WO-A2-2008/063902
- US-A- 5 118 792
- US-A- 5 998 200
- KIM YOUNG DUK ET AL: "Siloxane-based biocatalytic films and paints for use as reactive coatings" BIOTECHNOLOGY AND BIOENGINEERING, vol. 72, no. 4, 20 February 2001 (2001-02-20), pages 475-482, XP002535934 ISSN: 0006-3592
- ASURI P ET AL: "Polymer-nanotube-enzyme composites as active antifouling films" SMALL JANUARY 2007 WILEY-VCH VERLAG DE, vol. 3, no. 1, January 2007 (2007-01), pages 50-53, XP002535935
- VELICANGIL O ET AL: "PROTEASE COUPLED MEMBRANES FOR ULTRA FILTRATION" BIOTECHNOLOGY AND BIOENGINEERING, vol. 19, no. 12, 1977, pages 1891-1894, XP002535936 ISSN: 0006-3592
- Linqiu Cao: "3 Covalent Enzyme Immobilization" In: "Carrier-bound Immobilized Enzymes", 29 September 2005 (2005-09-29), Wiley-VCH Verlag GmbH & Co. KGaA, Weinheim, FRG, XP055099162, ISBN: 978-3-52-731232-0 pages 169-316, DOI: 10.1002/3527607668,
- DAVID ROBSON: 'Fish 'antifreeze' inspires ice-proof paint', [Online] 03 August 2007, Retrieved from the Internet: <URL:http://www.newscientist.com/article/dn 12420-fish-antifreeze-inspires-iceproof-pai nt.html> [retrieved on 2015-03-23]
- DR KONSTANTIN SIEGMANN ET AL: 'Anti-freeze Beschichtungen für Rotorblätter von Windenergieanlagen Schlussbericht Ausgearbeitet durch', [Online] 18 December 2006, XP055178611 Retrieved from the Internet: <URL:http://www.bfe.admin.ch/php/modules/en et/streamfile.php?file=000000009108.pdf&nam e=000000260051.pdf> [retrieved on 2015-03-23]

## Description

The present invention is directed to an object having an aero- or hydrodynamically active surface, wherein one or more types (families) of biocatalytic and/or anti-icing proteins are immobilized on its surface. The present invention is further directed a method of providing a self-cleaning and/or anti-freeze coating to an aero- or hydrodynamically active surface of an object.

In the prior art, there are several techniques available to provide "easy to clean" or self-cleaning surfaces of objects.

A first distinction can be made between techniques which do not require the addition of energy and those techniques, which are based on the use of energy.

The first group comprises the provision of artificial surface structures to objects which provide a self-cleaning effect to the surfaces of an object.

US 6,660,363 is directed to self-cleaning surfaces of objects having an artificial surface structure of elevations and depressions wherein the distances between said elevations and are in a predefined range, wherein at least the elevations consist of hydrophobic polymers or permanently hydrophobized materials and wherein said elevations can not be wetted by water or by water containing detergents.

US 2002/0016433 provides a coating composition for producing difficult-to-wet surfaces comprising a finely divided powder whose particles have a hydrophobic surface and a porous structure and one film-forming binder characterized by a certain surface tension. This process produces difficult-to-wet surfaces and provides for the use of the coating compositions for producing surfaces having a self-cleaning effect and for reducing the flow resistance for liquids in pipes.

US5118792 discloses the methods of improving freezing tolerance of organic materials using anti-freeze peptides.

WO96/30459 discloses the use of various peptides to inhibit ice crystal growth.

US 2004/0213904 describes a process for producing detachable dirt- and water-repellent surface coatings on articles. The process comprises suspending the hydrophobic particles in a solution of a silicon wax in a highly volatile siloxane and applying this suspension to at least one surface of the article, and then removing the highly volatile siloxane.

All of these processes and coatings have the disadvantage that no active degradation of organic materials is provided. Furthermore, the adherence of ice is only reduced, however, can not be avoided to a larger extent.

The second group of techniques involves the use of mechanical energy and/or the use of UV-radiation.

US Patent Application No. US 2006/0177371 discloses a method for preparing a gel containing nanometer titanium dioxide particles for visible light photocatalysis. The method comprises obtaining titanium hydroxide, converting titanium hydroxide into titanium dioxide by adding an oxidant, an improving agent, an optional acid and an optional surfactant to compose a solution; and aging the solution by heating to make the solution become a gel. The gel has photocatalytic characteristics and self-cleaning efficiency in the visible light range. The gel obtained from this method can be applied on surfaces of a substrate and has self-cleaning, photocatalytic and bactericidal properties when illuminated by visible light.

US 2004/0009119 provides a pyrogenic preparation of titanium dioxide, wherein a metal salt solution is atomized to form an aerosol which is injected into a production stream. The titanium dioxide may be used as a photocatalyst or as a UV absorber and may be used in the coating of glass or in plastics.

These approaches however require additional structural components and devices which have to be supplied with energy. This brings about a high effort in maintenance and increased technical complexity. In the photocatalytic approach, the degradation process is extremely slow and the degradation will not function in the absence of light.

EP1661955 discloses anti-fouling coatings comprising enzymes with anti-fouling activity.

The paper of Kim Young Duk et al., "Siloxane-based catalytic films and paints for use as reactive coatings", Biotechnology and Bioengineering, vol. 72, no. 4, 20 Feb. 2001 discloses reactive coatings comprising biocatalytic enzymes.

In view of the prior art cited, it is an object of the present invention to provide a self-cleaning surface of an object, in particular of an object having an aero- or hydrodynamically active surface, with self-cleaning activity that does not require the use or supply of energy. It is a further object of the present invention to provide a self-cleaning surface which is capable of removing or at least degrading the organic contaminations such as proteins, sugars and fats from surfaces and which furthermore has antifreeze or anti-icing characteristics.

It is a further object of the present invention to provide a surface of an object which does not require an ongoing regeneration and does not negatively influence aerodynamic or hydrodynamic characteristics of the surface and finally, does not require the use of organic solvents or surfactants.

These and further objects are solved by the subject-matter of the independent claims. Preferred embodiments are set forth in the dependent claims.

The present invention uses layers (surfaces) and objects, having self-cleaning characteristics which usually comprise a substrate (or matrix) and a thin film of immobilized organic macromolecules such as proteins. The function of those proteins is to degrade organic materials and remove it and/or to avoid the formation of ice on the surfaces.

It is one of the major advantages of the present invention that the proteins involved in this function are not consumed during the process of degradation due to their nature as biocatalytic agents. This means that the surface layer of proteins does not require any kind of regeneration and only small amounts of proteins (such as enzymes) are sufficient. A further advantage in this regard is that the aero- or hydrodynamically active surfaces have not to be covered completely by the proteins, but also a partial coverage and islets of proteins are sufficient in order to provide the effects of the present invention. As a consequence, the functional layers will work also in a case, in which already some parts thereof have been eroded or have been removed by other processes or have been damaged.

One additional advantage of the present invention is that due to the reduced thickness of the layers to be applied on the aero- or hydrodynamically active surface, the layers are transparent in the visible light and, thus, are perfectly suitable for finishing applications (i.e. the top most layer exposed to the environment) of windscreens, aircraft surfaces etc. A further important application of the modified surfaces of the invention are rotors of wind machines.

According to the invention, the biocatalytic and/or anti-icing proteins are applied to the surface of an object by a spacer (or linker) which positively contributes to the effects of the present invention since the protein confirmation is maintained and steric hindrance is avoided. This may result in an enzyme activity comparable to the activity in solution.

The present invention is in particular directed to the following aspects and embodiments:
In accordance with a first aspect of the present invention, there is provided an object having an aero- or hydrodynamically active surface, wherein one or more enzymes are immobilized on said surface by means of a cross-linker containing a spacer, and are coating said surface at least partially,
characterised in that:
one or more anti-icing proteins are also immobilized on said surface by means of a cross-linker containing a spacer, and are coating said surface at least partially; and
the aero- or hydrodynamically active surface is the surface of a wing of an aircraft.

As already mentioned above, the approach of the present invention has the great advantage that aero- or hydrodynamically active surfaces are not negatively influenced in their respective characteristics and thus is perfectly suitable for aero- or hydrodynamically active surfaces such as aircraft wings. The functional surface to be applied to the object usually is thin and its thickness ranges between about 10 nm and 1000 nm. It generally is transparent for visible and UV light.

It should be additionally noted that the groups of proteins (anti-icing proteins and enzymes) can be combined in order to fulfil their function in extreme environments (as they are required for example in aero- or hydrodynamically active surfaces of aircraft wings).

As already indicated above, one of the major advantages of the invention is that the coating of the enzymes and anti-icing proteins does not necessarily have to cover the complete surface of the object but it is sufficient that a partial coating (islets or spots) is applied in order to achieve the effects of the invention, i.e. to provide a self-cleaning surface on aero- or hydrodynamically active objects.

The enzymes may be selected from the group consisting of amylases, proteases, lipases, cellulases, nucleases, chitinases and mixtures thereof. The enzymes may be of natural origin or manufactured, for example, by chemical synthesis or by genetic engineering.

One of the usual applications of the present invention is the degradation of debris from insects, which adheres to the surface of an object. The body of an insect comprises nearly all conceivable organic materials such as sugars, fats, proteins etc. In order to remove and/or to degrade insect derived debris, a mixture of for example proteases, lipases and chitinases would be required. It is noted that the above list of enzymes of course is not limited and can be extended depending on the intended use of the object.

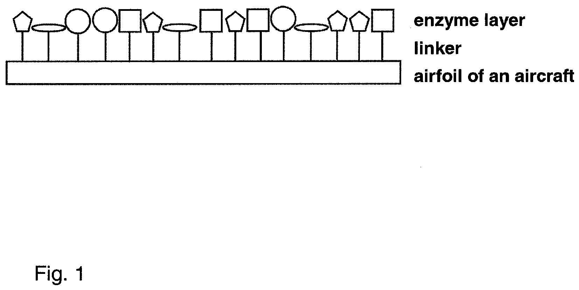

An illustration regarding the configuration of an enzyme layer immobilized to the surface of an object via a spacer is illustrated in Fig. 1.

According to a further embodiment, the anti-icing proteins are selected from antifreeze proteins (AFPs) of artificial or natural origin. Such natural AFPs might be derived from fish, insects or plants, in particular from *Pagothenia borchgrevinki, Eleginus gracilis,* - *Pseudepleuronectes americanus, Tenebrio molitor, or Choristoneura fumiferana*. Again, this list is not limited and can be extended based on new scientific developments and the specific requirements of the application. In addition, these proteins are not restricted to proteins of natural origin but also comprise artificial proteins, such as proteins manufactured and/or modified by recombination techniques, fusion proteins and the like.

In a further preferred embodiment, the surface to be coated is a micro- or nanostructured surface. Those micro- or nanostructured surfaces show improved aero- or hydrodynamic effects and my contribute to the reduction of flow resistance by means of specific geometries. It turned out that the aero- or hydrodynamic characteristics of those micro- or nanostructured surfaces are not negatively influenced by applying the proteins of the invention.

Further, micro- or nanostructured surfaces have the advantage to allow an improved adhesion of the proteins to the surface, which proteins might be less eroded and will maintain their function better.

In order to attach a protein to a surface, in most cases the surface will have to be activated first. In a preferred embodiment this modification will be done by applying a silane. In a preferred embodiment this modification will be done by applying a silane

If used, the silanes preferably are selected from the group of general formula 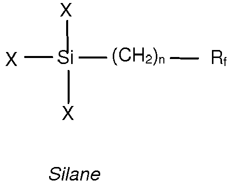 wherein
R_{f} = organofunctional group, preferably selected from amino, carboxyl, sulfhydryl, hydroxyl, cyano, epoxy, or aldehyde groups
n = an integer from 1-20
X = hydrolysable group, preferably methoxy, ethoxy, isopropoxy, or methoxyethoxy. It is noted that methoxy is preferred.

The further coupling reaction will be explained below:
1^{st} step: reacting a silane with the surface of the object ("activation") 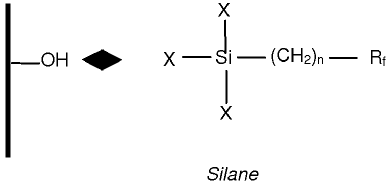 wherein
   R_{f} = organofunctional group, preferably selected from amino, carboxyl, sulfhydryl, hydroxyl, cyano, epoxy, aldehyde group;
   n = an integer from 1-20
   X = hydrolysable group, preferably methoxy; ethoxy; isopropoxy, methoxyethoxy;
   and
2^{nd} step: coupling the protein to the activated surface of the object via a crosslinker molecule 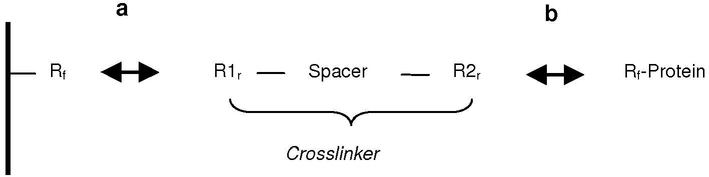
wherein the reactive groups R1ᵣ and R2ᵣ are the same (homobifunctional cross linkers) or different (heterobifunctional cross linkers) and are preferably independently selected from NHS-ester, maleimido, imido ester, carbodiimide, isocyanate, hydrazide groups.

It is noted that in this reaction, it is also possible to use silanes which are "dipodal", i.e. which carry 2 x 3 = 6 groups X and may thus result in 6 linkages with the substrate.

Furthermore, the following silanes might preferably be used in the first step:
Aminopropyltriethoxysilane (APTES)
Aminopropyltrimethoxysilane
Aminopropyldimethylethoxysilane
Aminohexylaminomethyltrimethoxysilane
Aminohexylaminopropyltrimethoxysilane
Triethoxysilylundecanal
Bis-2-Hydroxyethyl-3-aminopropyltriethoxysilane
Cyanopropyltrimethoxysilane
Mercaptopropyltrimethoxysilane
Epoxyhexyltriethoxysilane
Epoxypropoxytrimethoxysilane
Glycidoxypropyltrimethoxysilane (GOPS)
Octadecyltrimethoxysilane
Acryloxypropyltrimethoxysilane
Methacryloxypropyltrimethoxysilane.

The first step, i.e. the modification of the object's surface may also be replaced by coating the object with polyethylenimine or amino-PCP.

In the second step (i.e., attaching the protein to the activated surface of the object) with the help of a cross linker molecule, the following reactive groups R1ᵣ and/or R2ᵣ may be preferably used in the cross linker:

| R1ᵣ or R2ᵣ | reactive on R_{f}: | | | | |
|---|---|---|---|---|---|
| | - NH₂ | -SH | -COOH | -OH | -COH |
| | amine | sulfhydryls | carboxyls | hydroxyls | carbohydrates |
| NHS-ester | x | | | | |
| maleimide | | x | | | |
| imidoester | x | | | | |
| carbodiimide | x | | x | | |
| isocyanate | | | | x | |
| hydrazide | | | | | x |

On the protein side, the reaction partner R_{f} usually will be an amino or a carboxyl group.

Example for a coupling reaction via carbodiimide:

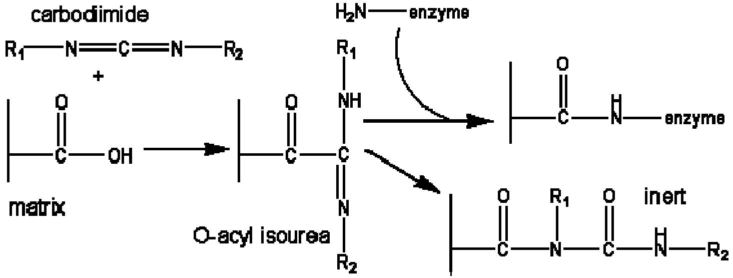

Example for a coupling reaction via cyanate:

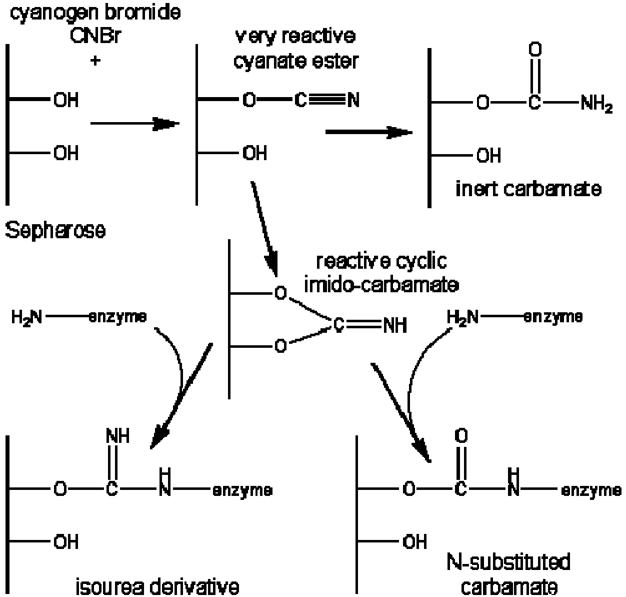

Preferred examples of crosslinkers are as follows:
- Ethyldimethylaminopropylcarbodiimide (heterobifunctional, amino+carboxyl reactive)
- Ethylendiisocyanate (homobifunctional, hydroxyl-reactive)
- Hexamethylendiisocyanate (homobifunctional, hydroxyl-reactive)
- Glutaraldehyde (homobifunctional, amino-reactive)

In a preferred embodiment, the reactive groups of the cross linker R1ᵣ and R2ᵣ are separated by a spacer. Examples for crosslinkers with a spacer group are:
- NHS-PEOₙ-maleimide (heterobifunctional, amino+sulfhydryl-reactive)
- Bis-NHS-PEOₙ (homobifunctional, amino-reactive)
- Bis-maleimide-PEOₙ (homobifunctional, sulfhydryl-reactive)
- Bis(sulfoNHS)suberate (homobifunctional, amino-reactive)
- Succinimidyl-maleimidophenyl-butyrate (homobifunctional, amino-reactive)

### (PEO = Polyethylenoxide; NHS- = Succinimidyl-)

The surface of the object may, in a further embodiment, preferably be coated by a polymeric coating, which serves as a spacer and as a repellent.

On the one hand, polymers provide a convenient kind of surface modification, on the other hand, they provide an enlarged surface, such that a larger amount of protein molecules can be bound. Due to the large distance of the bound protein (enzyme) to the surface of the object, the probability will increase that a correct protein folding and, thus, the function of the protein will be maintained.

Polymers may also act as a protein repellent, resulting in a coupling of desired proteins only, but not of "foreign" proteins. Last but not least, the polymer layers may also take the form of "hydrogels", i.e. three dimensional structures, which are capable of receiving taking up water or aqueous solutions. By this approach, an aqueous milieu is resulting locally, which is necessary for the function of most enzymes.

In the case, proteins shall be bound via such polymers to the surface of an object, the polymers have to be provided with reactive end groups (so called "capping"), e.g. with amino (-NH₂) or carboxyl (-COOH) groups. To these reactive end groups, proteins may be coupled by cross linking as described above.

In a preferred embodiment, the polymers are selected from one or more of the following classes:

### 1. Self-assembled monolayers (SAM; self-organizing-monolayers)

A self-assembled monolayer is formed spontaneously by immersing of surface active or organic substances in a solution or suspension. Suitable substances are for example chlorosilanes and alkylsilanes having a length of more than 10 carbon atoms. Those are forming highly ordered monolayers on gold, glass and silicon having a high inner order. Surfaces treated in this kind are stable in air for months. In contrast to conventional surface coatings, SAM's have a defined thickness in the range of 0,1 to 2 nm.

Examples of SAMs are:
Glycidoxypropyltrimethoxysilane
Trimethoxysilylpropylmethacrylate
PEG-PPG-PEG (PEG: Polyethylenglycol, PPG: Polypropylenglycol)

### 2. Star-shaped polymers

StarPEG is a star-shaped "prepolymer" having (in most cases 6) "arms" based on PEG. The ends (usually -OH) may be modified for example with isocyanate groups (-NCO), which in turn may react with primary amines (of proteins). Further end-modifications are acrylate and vinylsulfone-end groups.

The following options for coupling of proteins exist:
a) Binding proteins to StarPEG via isocyanate in solution prior to forming the layer (1 step coating);
b) Coupling to the isocyanate-group in fresh layers;
c) Coupling to amino-groups in already crosslinked layers.

### 3. Dendrimers

Dendrimers are highly branched "tree-shaped" polymer structures. Like linear polymers they may be provided with reactive end groups (so called "capping", see above). By means of these groups, they might be covalently bound to a surface. A bond to the surface is, however, also feasible by means of forming a film.

### Examples:

PAMAM (Polyamidoamine)-Dendrimer
Polylysine-Dendrimer

### 4. Polymer brushes

The term polymer brush is used for polymers adsorbed to a surface, that are tightly packed such that the individual polymer chains have to spread out from the substrate. End-functionalized polymers may be used in this respect to couple proteins (via crosslinker).

### Example:

Poly(DMA-b-GMA) (Block copolymer of dimethylacrylamide and glycidyl methacrylate)
Poly(hydroxyethylmethacrylate)
Poly(PEG)methacrylate

In a preferred embodiment, the proteins are coating about 20 to 100 % of the surface. As already indicated above, in many applications, it is sufficient to immobilize the proteins in a islet or spot like form on the surfaces of an object in order to fulfill the effects of the invention, i..e. degradation of organic materials and anti-freeze properties. However, based on technical experiences, it can be assumed that at least 25 % of the surface should be covered by enzymes and anti-icing proteins.

The present invention is also directed to a method of providing a self-cleaning and antifreeze coating to an aero- or hydrodynamically active surface of an aircraft surface, the method comprising of
a) providing one or more enzymes and anti-icing proteins; and
b) immobilizing the enzymes and anti-icing proteins to at least a part of the surface of the wing of the aircraft via a spacer.

In the method of the present invention, the proteins as indicated above can be used. The like, the spacers, surfaces etc. as indicated above will apply. Regarding the coupling reactions between proteins and the surface of the object, it is referred to the above information (see first aspect).

In a merely illustrative embodiment, the present invention is directed to the use of biocatalytic and/or anti-icing proteins for providing a self-cleaning and/or antifreeze coating to a surface of an object. This coating is suitable for removing organic materials from the surface of an object, in particular insects and debris of insects adhering to the surface of an object or algae and algae debris adhering to the underwater surface of a ship's hull.

Alternatively or in addition, the coating is suitable for avoiding the formation of ice on the surface of the object. Thus, the present invention is in particular suitable for providing a self-cleaning and/or anti-freeze surface to aircrafts.

The present invention now will be further illustrated by means of examples referring to the enclosed figures and drawings.

In the figures, the following is shown:
Fig. 1 is showing an embodiment of the invention, wherein proteins are coupled to the surface of a wing of an aircraft.

### Examples:

The following is a specific example of immobilizing an enzyme:
- Purifying of the object's surface (glass or titan)
- Applying a solution ofaminopropyltriethoxysilane
- Rinsing the excess
- Adding trypsine in coupling buffer (amine-free) to the surface
- Adding a solution of ethyldimethylaminopropylcarbodiimid
- Incubate for 30 min.at room temperature
- Rinsing
- Measuring the enzyme reaction on the surface by means of a clor reaction inphotometer.

Although the present invention has been illustrated by examples, it is not limited thereto but may be modified by a skilled person in any conceivable way.

## Claims

1. An object having an aero- or hydrodynamically active surface, wherein one or more enzymes are immobilized on said surface by means of a cross-linker containing a spacer, and are coating said surface at least partially,
**characterised in that**:
one or more anti-icing proteins are also immobilized on said surface by means of a cross-linker containing a spacer, and are coating said surface at least partially; and
the aero- or hydrodynamically active surface is the surface of a wing of an aircraft.

2. An object according to claim 1 wherein the enzymes are selected from the group consisting of amylases, proteases, lipases, cellulases, nucleases, chitinases and mixtures thereof, of natural and/or artificial origin.

3. An object according to claim 1 or claim 2 wherein the anti-icing proteins are selected from antifreeze proteins (AFP's) of artificial or natural origin.

4. An object according to claim 3 wherein the AFP is derived from fishes, insects or plants.

5. An object according to claim 4, wherein the AFP is derived from *Pagothenia borchgrevinki, Eleginus gracilis, Pseudopleuronectes americanus, Tenebrio molitor,* or *Choristoneura fumiferana*.

6. An object according to any preceding claim, wherein the surface has first been activated by applying silanes, wherein the silanes are selected from the group of general formula 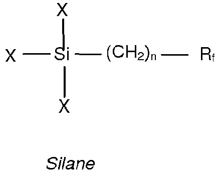 wherein
R_{f} = organofunctional group, preferably selected from amino, carboxyl, sulfhydryl, hydroxyl, cyano, epoxy, aldehyde -
n = an integer from 1-20
X = hydrolysable group, preferably methoxy; ethoxy; isopropoxy, methoxyethoxy.

7. An object according to any preceding claim, wherein the surface is coated by a polymeric coating, which serves as a spacer and as a repellent.

8. An object according to claim 7, wherein the surface is coated by self-assembled monolayers of polymers, such as glycidoxypropyltrimethoxysilane, trimethoxysilylpropylmethacrylate PEG-PPG-PEG (PEG: polyethylenglycol, PPG: polypropylenglycol), star shaped polymers, dendrimers or polymer brushes.

9. An object according to any preceding claim, wherein the proteins are coating about 25 % of the surface.

10. An object according to any preceding claim, wherein the proteins are coating about 50% of the surface.

11. An object according to any preceding claim, wherein the surface is micro- or nano structured.

12. An object according to any preceding claim, wherein the enzymes and the anti-icing proteins are immobilized on the surface in a spot-like manner or in islets.

13. A method of providing an object according to any preceding claim, the method comprising:
a) providing one or more enzymes and one or more anti-icing proteins; and
b) immobilizing the enzymes and anti-icing proteins to at least a part of the surface of the wing of the aircraft by means of a cross linker containing a spacer.

## Patentansprüche

1. Gegenstand mit aero- oder hydrodynamisch aktiver Oberfläche, wobei ein oder mehr Enzyme auf der Oberfläche mithilfe eines Vernetzungsmittels mit Abstandhalter immobilisiert sind und die Oberfläche zumindest teilweise bedecken,
**dadurch gekennzeichnet, dass**
ein oder mehr Einfrierschutzproteine auch auf der Oberfläche mithilfe eines Vernetzungsmittels mit Abstandhalter immobilisiert sind und die Oberfläche zumindest teils bedecken; und
die aero- oder hydrodynamisch aktive Oberfläche die Oberfläche eines Flügels eines Luftfahrzeugs ist.

2. Gegenstand nach Anspruch 1, wobei die Enzyme aus einer Gruppe, bestehend aus Amylasen, Proteasen, Lipasen, Cellulasen, Nukleasen, Chitinasen und Mischungen davon, natürlichen und/oder künstlichen Ursprungs, ausgewählt werden.

3. Gegenstand nach Anspruch 1 oder Anspruch 2, wobei die Einfrierschutzproteine aus Anti-Frost-Proteinen (AFP) natürlichen oder künstlichen Ursprungs ausgewählt werden.

4. Gegenstand nach Anspruch 3, wobei die AFP aus Fischen, Insekten oder Pflanzen gewonnen werden.

5. Gegenstand nach Anspruch 4, wobei die AFP aus *Pagothenia borchgrevinki, Eleginus gracilis, Pseudopleuronectes americanus, Tenebrio molitor* oder *Choristoneura fumiferana* gewonnen werden.

6. Gegenstand nach einem der vorherigen Ansprüche, wobei die Oberfläche erst durch die Anwendung von Silanen aktiviert wurde, wobei die Silane ausgewählt werden aus einer Gruppe der allgemeinen Formel: 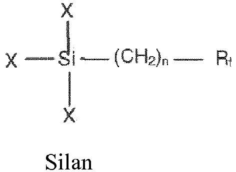 wobei
Rf = organofunktionelle Gruppe, bevorzugt ausgewählt aus Amino, Carboxyl, Sulfhydryl, Hydroxyl, Cyano, Epoxy, Aldehyd-
n = eine ganze Zahl zwischen 1 - 20
X = hydrolysierbare Gruppe, bevorzugt Methoxy; Ethoxy; Isopropoxy, Methoxyethoxy.

7. Gegenstand nach einem der vorherigen Ansprüche, wobei die Oberfläche von einer Polymerbeschichtung bedeckt ist, welche als Abstandhalter und Abwehrmittel wirkt.

8. Gegenstand nach Anspruch 7, wobei die Oberfläche von selbstorganisierenden Monoschichten aus Polymeren bedeckt ist, wie Glycidoxypropyltrimethoxysilan, Trimethoxysilylpropylmethacrylat PEG-PPG-PEG (PEG: Polyethylenglycol, PPG: Polypropylenglycol), sternförmige Polymere, Dendrimere oder Polymerbürsten.

9. Gegenstand nach einem der vorherigen Ansprüche, wobei die Proteine ca. 25 % der Oberfläche bedecken.

10. Gegenstand nach einem der vorherigen Ansprüche, wobei die Proteine ca. 50 % der Oberfläche bedecken.

11. Gegenstand nach einem der vorherigen Ansprüche, wobei die Oberfläche mikro- oder nanostrukturiert ist.

12. Gegenstand nach einem der vorherigen Ansprüche, wobei die Enzyme und die Einfrierschutzproteine auf der Oberfläche in einer fleckenförmigen Weise oder in Inseln immobilisiert sind.

13. Verfahren zur Bereitstellung eines Gegenstandes nach einem der vorherigen Ansprüche, wobei das Verfahren umfasst:
a. Bereitstellung von einem oder mehreren Enzymen und einem oder mehreren Einfrierschutzproteinen; und
b. Immobilisierung der Enzyme und Einfrierschutzproteine auf wenigstens einem Teil der Oberfläche des Flügels des Luftfahrzeugs mithilfe eines Vernetzungsmittels mit Abstandhalter.

## Revendications

1. Un objet présentant une surface active du point de vue aérodynamique ou du point de vue hydrodynamique, dans lequel un ou plusieurs enzymes sont immobilisés sur ladite surface par l'intermédiaire d'un agent de réticulation contenant un espaceur, lesquels enzymes revêtent au moins partiellement ladite surface,
**caractérisé en ce que**
une ou plusieurs protéines antigivrage sont également immobilisées sur ladite surface au moyen d'un agent de réticulation contenant un espaceur, lesquels enzymes revêtent au moins partiellement ladite surface, et
la surface active du point de vue aérodynamique ou du point de vue hydrodynamique est la surface d'une aile d'un aéronef.

2. Un objet selon la revendication 1, dans lequel les enzymes sont choisies dans le groupe consistant en amylases, protéases, lipases, cellulases, chitinases, nucléases d'origine naturelle et/ou artificielle et des mélanges de celles-ci,.

3. Un objet selon la revendication 1 ou 2, dans lequel les protéines antigivrage sont choisies parmi les protéines antigel (AFP) d'origine naturelle ou artificielle.

4. Un objet selon la revendication 3, dans lequel les AFP dérivent de poissons, d'insectes ou de plantes.

5. Un objet selon la revendication 4, dans lequel les AFP dérivent de *Pagothenia borchgrevinki, Eleginus gracilis, Pseudopleuronectes americanus, Tenebrio molitor ou Choristoneura fumiferana*.

6. Un objet selon une quelconque des revendications précédentes, dans lequel la surface a été préalablement activé par l'application de silanes, lesquels silanes sont choisis dans le groupe de formule générale 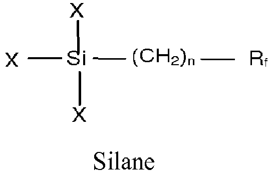 dans laquelle formule
R_{f} = groupe organofonctionnel, de préférence choisi parmi les groupes amino, carboxyle, sulfhydryle, hydroxyle, cyano, époxy, aldéhyde -
n = un nombre entier de 1 à 20
X = un groupe hydrolysable, de préférence méthoxy; éthoxy; isopropoxy, méthoxyéthoxy.

7. Un objet selon une quelconque des revendications précédentes, dans lequel la surface est recouverte d'un revêtement polymère, qui sert d'espaceur et de répulsif.

8. Un objet selon la revendication 7, dans lequel la surface est revêtue de monocouches auto-assemblées de polymères, tels que le glycidoxypropyltriméthoxysilane, le triméthoxysilylpropylméthacrylate, PEG-PPG-PEG (PEG: = polyéthylène glycol, PPG = polypropylèneglycol), les polymères en forme d'étoile, les dendrimères ou des brosses polymères.

9. Un objet selon une quelconque des revendications précédentes, dans lequel les protéines recouvrent environ 25% de la surface.

10. Un objet selon une quelconque des revendications précédentes, dans lequel les protéines recouvrent environ 50% de la surface.

11. Un objet selon une quelconque des revendications précédentes, dans lequel la surface est micro structurée ou nanostructurée.

12. Un objet selon une quelconque des revendications précédentes, dans lequel les enzymes et les protéines antigivrage sont immobilisées sur la surface de façon ponctuelle ou en ilots.

13. Un procédé d'obtention d'un objet selon une quelconque des revendications précédentes, ce procédé comprenant:
a) la fourniture de un ou plusieurs enzymes et de une ou plusieurs protéines antigivrage; et
b) l'immobilisation des enzymes et des protéines antigivrage sur au moins une partie de la surface de l'aile d'un aéronef au moyen d'un agent de réticulation contenant un espaceur.
